# EUROPÄISCHE PATENTANMELDUNG

(11) **EP 4 378 655 A1**
(43) Veröffentlichungstag der Anmeldung: **05.06.2024**
(21) Anmeldenummer: 23210312.7
(22) Anmeldetag: 16.11.2023
(51) Int. Cl.: B29C 35/02, B29C 71/02, B29K 21/00, G01N 33/44

(54) **VERFAHREN ZUR THERMISCHEN BEHANDLUNG EINES VORVERNETZTEN ELASTOMER-BAUTEILS**

(30) Priorität: 30.11.2022 DE 102022131690
(71) Anmelder: Carl Freudenberg KG, 69469 Weinheim (DE)
(72) Erfinder: Peter, David, 67122 Altrip (DE); Neumann, Christopher, 64686 Lautertal (DE); Bittner, Denis, 65474 Bischofsheim (DE); Opdenwinkel, Kai, 67661 Kaiserslautern (DE); Gerding, Lars, North Andover (Massachusetts), 01845 (US); Reichert, Peter, 55283 Nierstein (DE)

(57) **Zusammenfassung**

Die Erfindung betrifft Verfahren zur thermischen Behandlung mindestens eines vorvernetzten Elastomer-Bauteils, wobei die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert, während der thermischen Behandlung gemessen wird und die erhaltenen Messwerte zur Einstellung von mindestens einem Verfahrensparameter verwendet werden.

## Beschreibung

### Technisches Gebiet

Die Erfindung betrifft ein Verfahren zum Nachvernetzen von Elastomer-Bauteilen, sowie ein mit dem Verfahren hergestelltes Elastomer-Bauteil.

### Stand der Technik

Bei der Herstellung von Elastomer-Bauteilen ist eine thermische Behandlung in Form eines Nachheizens ein wichtiger Prozess, der die gewünschte Funktionalität der Produkte gewährleistet. Während des Nachheizens findet in der Regel eine Nachvernetzung des Elastomers statt. Wichtige Bauteile sind beispielsweise Flüssigsilikon-Bauteile (Liquid Silicone Rubber-Compounds/LSR-Compounds), weil hier sehr feine und präzise Formteile für verschiedene Industrien, wie Medizintechnik, Lebensmittel- und Pharmazieindustrie sowie Elektrotechnik erhalten werden können. Flüssigsilikon-Bauteile werden nach der Formgebung auf Basis vorgegebener Spezifikationen, für einen bestimmten Zeitraum und bei einer bestimmten Temperatur, wie z.B. 4 Stunden bei 200°C, thermisch nachbehandelt. Die thermische Nachbehandlung dient dazu die mechanischen Eigenschaften der Bauteile auf die gewünschten Werte einzustellen. Im Weiteren dient sie auch dazu flüchtige Bestandteile, sogenannte Volatile Organic Compounds (VOCs), z. B. niedrigsiedende Abspaltprodukte aus der Vernetzungsreaktion, aus dem Material / Bauteil zu entfernen.

Im Stand der Technik beschreibt die WO1998029787A1 eine Prozessanlage zur Herstellung von Halobutylkautschuk, die mit einer online Überwachung und Steuerung ausgestattet ist, die die Prozess-Parameter kontrolliert. Durch ein enthaltenes in-situ-Messsystem müssen für Messungen keine Proben aus dem Prozess entnommen werden. Es wird ein Fourier-Transformations-Nahinfrarot (FTNIR)-Spektrometer, Glasfaserkabel, ein Viskosimeter zur Messung der Lösungsviskosität und ein Widerstandstemperaturgerät (RTD) zur Temperaturmessung verwendet. Ein online Echtzeit-Analysator-System, das ein CPCA-Analyse (Constrained Principal Spectral) Programm verwendet, sagt die Eigenschaft des Polymerprodukts voraus und versorgt das Prozesssteuerungssystem mit einer Analyse der Daten unter Verwendung abgeleiteter Beziehungen zwischen den physikalischen Eigenschaften des Polymers, den Spektralmessungen und den gemessenen Flüssigkeitsviskositäts- und Temperaturwerten. Die Prozess-Parameter werden über die Unterschiede zwischen den vorhergesagten und gewünschten Eigenschaften des Produkts gesteuert. Das in dieser Druckschrift beschriebene Verfahren ist für die Nachvernetzung von Elastomer-Bauteilen nicht geeignet, da erfindungsgemäß die Formgebung schon stattgefunden hat und das Bauteil als festes Produkt vorliegt. Die für das oben beschriebene Verfahren eingesetzte Bestimmung der physikalischen Eigenschaften erfolgt dagegen in flüssiger, zum Teil hochviskoser Phase, die eine Homogenisierung durch entsprechendes Rühren erfordert. Eine Anwendung im Batchverfahren kann ebenfalls nicht erfolgen.

Die WO1991007650A1 beschreibt ein Verfahren zur Messung des Härtungsgrades eines Harzes in einem Verbundwerkstoff, wie z.B. vorimprägnierten Fasern, sogenannten Prepreg (preimpregnated fibres). Eine wichtige Anforderung des Herstellungsverfahrens jeder aus Prepregs gebildeten Laminatstruktur ist die Bestimmung der Menge oder des Härtungsgrads des Polymerharzes. Hierzu wird eine Infrarotspektrometervorrichtung zur Auflösung spezifischer Frequenzabsorptionsinformationen und zur Bereitstellung einer quantitativen Spektralanalyse eingesetzt. Die Menge an adsorbierter Energie bei einer ersten und zweiten Frequenz der Strahlung ist hierbei charakteristisch für unvernetzte und vernetzungsreaktive Gruppen des Harzes. Die beschriebene Methode umfasst die Bestrahlung des Verbundwerkstoffs mit einer Infrarot-Lichtquelle, die Erzeugung einer quantitativen Spektralanalyse der bei der ersten und zweiten Frequenz absorbierten Energiemenge, die Bestimmung der Höhe oder der Fläche unter dem Maximum des Spektrums bei jeder ersten und zweiten Frequenz, sowie der Berechnung eines Verhältnisses zwischen einem der bestimmten Werte und dem anderen Wert, um ein Maß für den Härtungsgrad des Harzes zu erhalten. Nachteilig an diesem Verfahren ist, dass nur die Eigenschaften einzelner Bauteile gemessen werden. Darüber hinaus ist das Verfahren für die Nachvernetzung von Elastomer-Bauteilen nicht geeignet, da die Konzentrationsunterschiede funktioneller Gruppen vorvernetzer Bauteile gegenüber getemperten Bauteilen in der Regel sehr gering sind.

### Aufgabenstellung

Die Aufgabe der hier vorliegenden Erfindung ist es ein Verfahren zur thermischen Behandlung eines vorvernetzten Elastomer-Bauteils bereitzustellen, welches es ermöglicht verschiedene Verfahrensparameter, beispielsweise die Dauer der thermischen Behandlung, die Luftwechselrate und/oder den Temperaturverlauf, während der thermischen Behandlung zu optimieren, ohne die Behandlung unterbrechen zu müssen. Dies ermöglicht Energieeinsparungen und eine Reduktion des CO₂-Ausstosses bei gleichzeitiger Einstellung der gewünschten mechanischen und/oder physikalischen Eigenschaften, so dass ein insgesamt nachhaltiges Verfahren bereitgestellt wird.

### Technische Lösung

Gelöst wird diese Aufgabe durch ein Verfahren zur thermischen Behandlung mindestens eines vorvernetzten Elastomer-Bauteils, in dem die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil während der thermischen Behandlung korreliert, gemessen wird und die erhaltenen Messwerte zur Einstellung von mindestens einem Verfahrensparameter verwendet werden.

Erfindungsgemäß wurde erkannt, dass es vorteilhaft ist, die flüchtigen Abspaltprodukte der Nachvernetzungsreaktion von vorvernetzten Elastomer-Bauteilen und insbesondere ihre Konzentrationsänderung zu bestimmen, da dies einen direkten Rückschluss auf wesentliche Performance-Parameter des Bauteils im Prozess, beispielsweise den Umsetzungsgrad der Nachvernetzungsreaktion des Elastomer-Bauteils, mechanische Eigenschaften des Bauteils, den Restgehalt flüchtiger Bestandteile im Elastomer-Bauteil und/oder den Restgehalt extrahierbarer Bestandteile im Elastomer-Bauteil ermöglicht. In einer Ausführungsform ist der Performance-Parameter ausgewählt unter dem Umsetzungsgrad der Nachvernetzungsreaktion des Elastomer-Bauteils und/oder mechanischen Eigenschaften des Bauteils. Unter dem Begriff Abspaltprodukte sollen erfindungsgemäß nicht nur Substanzen verstanden werden, die chemisch von dem Elastomer-Bauteil abgespalten werden sondern auch Substanzen, die im Bauteil lediglich physikalisch gebunden vorliegen und während der thermischen Behandlung freigesetzt werden. Bevorzugte Abspaltprodukte sind Substanzen, die chemisch von dem Elastomer-Bauteil abgespalten werden.

Das erfindungsgemäße Verfahren hat zudem noch sicherheitsrelevante Vorteile, da während der thermischen Behandlung eine Aussage über die Konzentrationsentwicklung der Abspaltprodukte getroffen werden kann und bei kritischen Konzentrationen, beispielweise bei der Bildung explosionsfähiger Gemische, die Luftwechselrate erhöht und/oder die Behandlung abgebrochen werden kann. Darüber hinaus ermöglicht das erfindungsgemäße Verfahren eine zumindest für den Betrieb zerstörungsfreie Verfahrensführung. In einer bevorzugten Ausführungsform umfasst das erfindungsgemäße Verfahren mithin mindestens ein zerstörungsfreies Betriebsverfahren.

Vorzugsweise werden die flüchtigen Abspaltprodukte so ausgewählt, dass ihre Konzentration und/oder ihre Konzentrationsänderung einen Rückschluss auf mindestens einen Performance-Parameter des Elastomer-Bauteils ermöglicht, vorzugsweise auf den Umsetzungsgrad der Nachvernetzungsreaktion des Elastomer-Bauteils, auf mechanische Eigenschaften des Bauteils, auf den Restgehalt flüchtiger Bestandteile im Elastomer-Bauteil und/oder auf den Restgehalt extrahierbarer Bestandteile im Elastomer-Bauteil. In einer Ausführungsform umfasst der mindestens eine Performance-Parameter des Elastomer-Bauteils den Umsetzungsgrad der Nachvernetzungsreaktion des Elastomer-Bauteils und/oder mechanische Eigenschaften des Bauteils.

In einer bevorzugten Ausführungsform findet das Messen der Änderung des mindestens einen Messparameters inline und/oder online statt. Ganz besonders bevorzugt findet die Messung der Änderung des Messparameters ohne Unterbrechung des Verfahrens statt.

Unter einer online Bestimmung versteht man, dass nicht direkt im Prozess, sondern in einer eingebauten Abzweigung, beispielsweise einem Bypass, die Probe vermessen wird. Inline wird die Probe direkt im Prozess vermessen, die Messstelle befindet sich direkt am Reaktionsort. Vorteilhaft ist in beiden Fällen, dass der Prozess der thermischen Behandlung nicht unterbrochen werden muss.

Unter einem vorvernetzten Elastomer-Bauteil versteht man ein Bauteil, das eine teilweise vernetzte elastomere Komponente aufweist, die jedoch noch vernetzbare Gruppen aufweist, so dass das Bauteil durch thermische Behandlung noch weiter vernetzt werden kann. Im Folgenden wird das vorvernetzte Elastomer-Bauteil auch einfach Elastomer-Bauteil genannt.

Erfindungsgemäß weist das Elastomer-Bauteil mindestens ein elastomeres Material auf, bei dessen thermischer Behandlung flüchtige Abspaltprodukte entstehen können. In einer bevorzugten Ausführungsform weist das Elastomer-Bauteil mindestens ein elastomeres Material ausgewählt aus der Gruppe bestehend aus Siloxanen, vorzugsweise Polysiloxanen und noch bevorzugter Silikonen, insbesondere Liquid Silicon Rubber (LSR), Fluorkautschuk (FKM), Polyacrylatkautschuk (ACM), deren Copolymeren und/oder Blends hiervon auf. Dabei liegen die vorgenannten elastomeren Materialien im Elastomer-Bauteil vorvernetzt vor. Der Anteil der vorgenannten elastomeren Materialien, bezogen auf das Gesamtgewicht des Elastomer-Bauteils, beträgt vorzugsweise mindestens 5 Gew.%, beispielsweise von 5 Gew.% bis 100 Gew.%, noch bevorzugter mindestens 50 Gew.%, beispielsweise von 50 Gew.% bis 100 Gew.%, insbesondere von 70 Gew.% bis 100 Gew.%. In einer besonders bevorzugten Ausführungsform weist das Elastomer-Bauteil mindestens Siloxan als elastomeres Material auf.

Es ist denkbar, dass das Elastomer-Bauteil nur aus elastomeren Komponenten besteht. Denkbar ist aber auch, dass das Elastomer-Bauteil elastomere und nicht elastomere Komponenten, beispielsweise metallische Komponenten, nicht elastomere Polymere, insbesondere Thermoplaste und/oder Duroplaste, aufweist. Eine bevorzugte metallische Komponente ist Edelstahl. Ein bevorzugter Thermoplast ist Polyester.

In einer bevorzugten Ausführungsform liegt das Elastomer-Bauteil bei der Durchführung des Verfahrens in einem festen Aggregatzustand vor. Darüber hinaus liegt das Elastomer-Bauteil bevorzugt bei 25°C und einem Druck von 1 bar im festen Aggregatzustand vor.

Bevorzugte Abspaltprodukte, die bei der thermischen Behandlung des Elastomer-Bauteils freigesetzt werden, weisen Si-O- und/oder Si-C- Gruppen als funktionelle Gruppen auf. Dies gilt insbesondere für den bevorzugten Fall, dass das Elastomer-Bauteil Siloxane aufweist. Bevorzugte Abspaltprodukte weisen ferner spezifische Absorptionsbanden im Wellenzahlbereich von 4000 cm^-1 bis 400 cm^-1 auf. Besonders bevorzugte Abspaltprodukte sind Silikonabspaltprodukte, insbesondere Dodecamethylcyclohexasiloxan (D6), Decamethylcyclopentasiloxan (D5) und/oder Octamethylcyclotetrasiloxan (D4). Weitere bevorzugte Abspaltprodukte sind Kohlenstoffmonoxid, Schwefeldioxid, Stickstoffdioxid, Kohlenstoffdioxid, Chlorwasserstoff und/oder Fluorwasserstoff. Diese Abspaltprodukte entstehen in der Regel aus Fluorkautschuk (FKM) und/oder Polyacrylatkautschuk (ACM).

Mit dem erfindungsgemäßen Verfahren können unter Verwendung der erhaltenen Messwerte zur Änderung des mindestens einen Messparameters verschiedene Verfahrensparameter eingestellt werden. Besonders geeignete Verfahrensparameter sind die Dauer der thermischen Behandlung, die Luftwechselrate und/oder der Temperaturverlauf. In einer bevorzugten Ausführungsform sind die Verfahrensparameter die Luftwechselrate und/oder der Temperaturverlauf. Dabei erfolgt die Einstellung des mindestens einen Verfahrensparameters bevorzugt so, dass die Verfahrensführung verbessert und/oder optimiert wird.

In einer bevorzugten Ausführungsform werden die erhaltenen Messwerte zur Einstellung der Luftwechselrate und/oder des Temperaturverlaufs für das Verfahren, insbesondere für mindestens ein Betriebsverfahren verwendet.

In einer bevorzugten Ausführungsform werden die erhaltenen Messwerte wie folgt zur Einstellung des mindestens einen Verfahrensparameters verwendet:
A1) Die erhaltenen Messwerte werden in mindestens einem Kalibrierverfahren zur Ermittlung einer Grenzkonzentration der flüchtigen Abspaltprodukte verwendet, deren Überschreiten einen unerwünschten Einfluss auf das Verfahren hat, und
A2) in mindestens einem Betriebsverfahren wird die Luftwechselrate erhöht und/oder dieTemperatur verringert, sobald die Konzentration der flüchtigen Abspaltprodukten einen Wert erreicht, der in einem festgelegten Abstand von der Grenzkonzentration liegt,
   und/oder
B1) die erhaltenen Messwerte werden in mindestens einem Kalibrierverfahren zur Ermittlung eines optimierten Luftwechselratenverlaufs verwendet und
B2) in mindestens einem Betriebsverfahren wird der optimierte Luftwechselratenverlauf eingestellt;
   und/oder
C1) die erhaltenen Messwerte werden in mindestens einem Kalibrierverfahren zur Ermittlung eines optimierten Temperaturverlaufs verwendet und
C2) der optimierte Temperaturverlauf wird in mindestens einem Betriebsverfahren eingestellt.

In einer bevorzugten Ausfürhrungsform werden die erhaltenen Messwerte wie folgt zur Einstellung des mindestens einen Verfahrensparameters verwendet:
A1) Die erhaltenen Messwerte werden in mindestens einem Kalibrierverfahren zur Ermittlung der Grenzkonzentration der flüchtigen Abspaltprodukte verwendet, deren Überschreiten einen unerwünschten Einfluss auf das Verfahren hat, und
A2) wenn die Konzentration der flüchtigen Abspaltprodukten einen Wert erreicht, der in einem festgelegten Abstand von der Grenzkonzentration liegt, wird die Luftwechselrate in mindestens einem Betriebsverfahren erhöht und/oder dieTemperatur verringert.

Ein unerwünschten Einfluss auf das Verfahren ist beispielsweise die Ausbildung einer entzündbaren Atmosphäre, insbesondere im Reaktor und/oder die Bildung unerwünschter Reaktionsprodukte im Reaktor, eine zu hohe Schadstoffkonzentration im Reaktor und mithin in der Abluft und/oder eine Überschreitung von Emissionsgrenzwerten. Bevorzugt ist ein unerwünschter Einfluss die Ausbildung einer Atmosphäre im Reaktor, bei dem einer oder mehrere der folgenden Emissionsgrenzwerte überschritten werden: Für Kohlenstoffmonoxid 500 mg/m³, Schwefeldioxid 500 mg/m³, Stickstoffdioxid 500 mg/m³, Chlorwasserstoff 30 mg/m³, Fluorwasserstoff 5 mg/m³ und/oder für den Gesamtgehalt an flüchtigen organischen Verbindungen VOC 1500 mg C/m³. Flüchtige organische Verbindungen sind erfindungsgemäß organische Verbindungen mit einem Siedebereich von 50 bis 260 °C bei 1 Atmosphäre.

Die erhaltenen Messwerte sind bevorzugt die Änderung mindestens eines Messparameters, der mit der Konzentration von flüchtigen Abspaltprodukten aus dem Elastomer-Bauteil korreliert. Aus den erhaltenen Messwerten kann mithin auf die Konzentration der flüchtigen Abspaltprodukten geschlossen werden.

Der festgelegte Abstand von der Grenzkonzentration kann auch Null sein. Bevorzugt ist der festgelegte Abstand von der Grenzkonzentration jedoch ungleich Null. Vorzugsweise wird der Abstand so festgelegt, dass der unerwünschte Einfluss auf das Verfahren vermieden werden kann.

Weiter bevorzugt wird die Luftwechselrate in mindestens einem Betriebsverfahren A2) erhöht und/oder dieTemperatur verringert, wenn die Konzentration der flüchtigen Abspaltprodukten einen Wert erreicht, der in einem festgelegten Abstand unterhalb der Grenzkonzentration liegt.

In einer weiteren bevorzugten Ausführungsform werden die erhaltenen Messwerte wie folgt zur Einstellung des mindestens einen Verfahrensparameters verwendet:
B1) die erhaltenen Messwerte werden in mindestens einem Kalibrierverfahren zur Ermittlung eines verbesserten Luftwechselratenverlaufs verwendet und
B2) in mindestens einem Betriebsverfahren wird der verbesserte Luftwechselratenverlauf eingestellt.

Die erhaltenen Messwerte können die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert, sein.

Der verbesserte Luftwechselratenverlauf ist in einer Ausführungsform ein Luftwechselratenverlauf, bei dem die Luftwechselrate möglichst gering ist und die Atmosphäre, insbesondere im Reaktor, dennoch keinen unerwünschten Einfluss auf das Verfahren hat, beispielsweise nicht entzündlich ist, keinen negativen Einfluss auf die Perfomance des Elastomer-Bauteils hat, nicht zu einer zu hohen Schadstoffkonzentration im Reaktorund mithin in der Abluft und/oder nicht zu einer Überschreitung von Emissionsgrenzwerten führt. Die Emissionsgrenzwerte sind dabei bevorzugt, wie oben definiert.

Wird beispielsweise in mindestens einem Kalibrierverfahren festgestellt, dass sich bei einem Luftwechselratenverlauf an einem bestimmten Zeitpunkt eine zu hohen Schadstoffkonzentration im Reaktor bildet, wird für den verbesserten Luftwechselratenverlauf des mindestens einen Betriebsverfahrens die Luftwechselrate zumindest für diesen Zeitpunkt höher eingestellt.

Bevorzugt wird auch im Betriebsverfahren B2) die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert, gemessen und der Luftwechselratenverlauf B2) anhand der Messwerte weiter verbessert. Die Verbesserung kann darin bestehen, dass die Messwerte im Betriebsverfahren mit den korrespondierenden Messwerten des im Kalibrierverfahren ermittelten Verfahrens mit dem verbesserten Luftwechselratenverlaufs verglichen werden, und der Luftwechselratenverlauf B2) so angepasst wird, dass die Messwerte im Betriebsverfahren sich den Messwerten des im Kalibrierverfahren ermittelten Verfahrens mit dem verbesserten Luftwechselratenverlaufs annähern.

In einer weiteren bevorzugten Ausführungsform werden die erhaltenen Messwerte wie folgt zur Einstellung des mindestens einen Verfahrensparameters verwendet:
C1) die erhaltenen Messwerte werden in mindestens einem Kalibrierverfahren zur Ermittlung eines verbesserten Temperaturverlaufs verwendet und
C2) der verbesserte Temperaturverlauf wird in mindestens einem Betriebsverfahren eingestellt.

Der verbesserte Temperaturverlauf ist in einer Ausführungsform ein Temperaturverlauf, bei dem die Temperatur der thermischen Behandlung und/oder die Dauer der thermischen Behandlung möglichst gering ist und die thermische Behandlung des vorvernetzten Elastomer-Bauteils dennoch in erwünschter Weise abläuft.

Der verbesserte Temperaturverlauf ist in einer Ausführungsform ein Temperaturverlauf, bei dem sowohl die Temperatur der thermischen Behandlung als auch die Dauer der thermischen Behandlung möglichst gering sind und die thermische Behandlung des vorvernetzten Elastomer-Bauteils dennoch in erwünschter Weise abläuft.

In einer weiteren Ausführungsform ist ein verbesserte Temperaturverlauf ein Temperaturverlauf, bei dem die Temperatur der thermischen Behandlung möglichst hoch ist aber die Atmosphäre, insbesondere im Reaktor, dennoch keinen unerwünschten Einfluss auf das Verfahren hat, beispielsweise nicht entzündlich ist, keinen negativen Einfluss auf die Perfomance des Elastomer-Bauteils hat nicht zu einer zu hohen Schadstoffkonzentration im Reaktor und mithin in der Abluft und/oder nicht zu einer Verletzung von Emissionsgrenzwerten führt. Die Emissionsgrenzwerte sind dabei bevorzugt, wie oben definiert.

Bevorzugt wird auch im Betriebsverfahren C2) die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert, gemessen und der Temperaturverlauf B2) anhand der Messwerte weiter verbessert. Die Verbesserung kann darin bestehen, dass die Messwerte im Betriebsverfahren mit den korrespondierenden Messwerten des im Kalibrierverfahren ermittelten Verfahrens mit dem verbesserten Temperaturverlauf verglichen werden, und der Temperaturverlauf C2) so angepasst wird, dass die Messwerte im Betriebsverfahren sich den Messwerten des im Kalibrierverfahren ermittelten Verfahrens mit dem verbesserten Temperaturverlauf annähern.

Ein wesentlicher Schritt im erfindungsgemäßen Verfahren ist die Ermittlung der Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert. Dabei werden die flüchtigen Abspaltprodukte bevorzugt so ausgewählt, dass ihre Konzentration und/oder die Änderung ihrer Konzentration einen Rückschluss auf mindestens einen Performance-Parameter des Elastomer-Bauteils ermöglicht, vorzugsweise auf den Umsetzungsgrad der Nachvernetzungsreaktion des Elastomer-Bauteils, auf mechanische Eigenschaften des Bauteils, auf den Restgehalt flüchtiger Bestandteile im Elastomer-Bauteil und/oder auf den Restgehalt extrahierbarer Bestandteile im Elastomer-Bauteil.

Bevorzugte Messparameter sind ausgewählt aus der Konzentration der freigesetzten Abspaltprodukte, der Änderung der Konzentration der freigesetzten Abspaltprodukte, dem Flächeninhalt einer IR-Absoptionsbande der freigesetzten Abspaltprodukte und/oder der Intensität einer IR-Absoptionsbande der freigesetzten Abspaltprodukte.

Weiter bevorzugt umfasst das erfindungsgemäße Verfahren ein Kalibrierverfahren und ein Betriebsverfahren. Mit dem Kalibrierverfahren wird vorzugsweise mindestens ein Messparameter-Sollwert festgelegt, der mit mindestens einem Soll-Wert des Performance-Parameters korreliert. So kann mit dem Kalibrierverfahren ein Messparameter-Sollwert festgelegt werden, der einen Rückschluss auf einen Performance-Parameter des Elastomer-Bauteils ermöglicht, vorzugsweise auf den Umsetzungsgrad der Nachvernetzungsreaktion des Elastomer-Bauteils, auf mechanische Eigenschaften des Bauteils, auf den Restgehalt flüchtiger Bestandteile im Elastomer-Bauteil und/oder auf den Restgehalt extrahierbarer Bestandteile im Elastomer-Bauteil.

Dabei wird der Messparameter-Sollwert vorzugsweise durch kontinuierlichen oder in festgelegten Zeitabständen stattfindenden Abgleich der Ist-Werte des Performance-Parameters eines Elastomer-Testbauteils mit dem Soll-Wert des Performance-Parameters des Elastomer-Testbauteils sowie dem hierzu korrelierenden Messparameter-Istwert ermittelt.

Das Betriebsverfahren dient vorteilhafterweise zur thermischen Behandlung des vorvernetzten Elastomer-Bauteils, wobei während der thermischen Behandlung vorzugsweise ein Vergleich der Ist-Konzentration der freigesetzten Abspaltprodukte mit ihrer im Kalibrierverfahren ermittelten Soll-Konzentration, ein Vergleich der Ist-Änderung der Konzentration von flüchtigen Abspaltprodukten mit ihrer im Kalibrierverfahren ermittelten Soll-Änderung, ein Vergleich des Ist-Flächeninhalts einer IR-Absoptionsbande der freigesetzten Abspaltprodukte mit ihrem im Kalibrierverfahren ermittelten Soll-Flächeninhalt und/oder ein Vergleich der Ist-Intensität einer IR-Absoptionsbande der freigesetzten Abspaltprodukte mit ihrer im Kalibrierverfahren ermittelten Soll-Intensität erfolgt, und dieser Vergleich zur Einstellung von Verfahrensparametern verwendet wird.

Wie oben dargelegt umfassen bevorzugte Verfahrensparameter die Dauer der thermischen Behandlung, die Luftwechselrate und/oder den Temperaturverlauf. In einer Ausführungsform sind bevorzugte Verfahrensparameter die Luftwechselrate und/oder der Temperaturverlauf. In einer Ausführungsform sind bevorzugte Verfahrensparameter die Luftwechselrate, der Temperaturverlauf und/oder der Energieverbrauch des Verfahrens zur thermischen Behandlung, insbesondere des Betriebsverfahrens. Das erfindungsgemäße Verfahren ermöglicht mithin eine Optimierung von insbesondere Verfahrensdauer, Luftwechselrate und/oder Temperaturverlauf, so dass eine Energieeinsparung und eine Reduktion des Energieverbrauchs und CO₂-Ausstoßes erreicht werden kann. Ein weiterer bevorzugter Verfahrensparameter ist der Energieverbrauch des Verfahrens zur thermischen Behandlung, insbesondere des Betriebsverfahrens.

Vorzugsweise werden zunächst ein oder mehrere Kalibrierverfahren und anschließend ein oder bevorzugt mehrere Betriebsverfahren durchgeführt. Dabei werden die einzelnen Verfahrensschritte des Kalibrierverfahrens und des Betriebsverfahrens unabhängig voneinander vorzugsweise zumindest zum Teil nacheinander durchgeführt. In einer weiteren bevorzugten Ausführungsform werden mindestens zwei Kalibrierverfahren durchgeführt, wobei die Behandlung des Testbauteils bei verschiedenen Temperaturen durchgeführt wird. Dies ermöglicht es ein optimales Temperaturprofil für das Betriebsverfahren zu ermitteln.

Der im Kalibrierverfahren ermittelte Messparameter-Sollwert wird vorzugsweise so festgelegt, dass er einen Zeitpunkt widerspiegelt, bei dem mindestens ein Performance-Parameter des Testbauteils seinen Sollwert erreicht. Geeignete Performance-Parameter sind beispielsweise der Umsetzungsgrad der Nachvernetzungsreaktion des Testbauteils, mechanische Eigenschaften des Bauteils, der Restgehalt flüchtiger Bestandteile im Testbauteil und/oder der Restgehalt extrahierbarer Bestandteile im Testbauteil. In einer Ausführungsform ist der Performance-Parameter ausgewählt aus dem Umsetzungsgrad der Nachvernetzungsreaktion des Testbauteils und/oder mechanischen Eigenschaften des Bauteils.

Mit dem Kalibrierverfahren wird vorzugsweise ein Messparameter-Sollwert bestimmt, vorzugsweise der Sollwert für die Konzentration der freigesetzten Abspaltprodukte, der Sollwert für die Änderung der Konzentration der freigesetzten Abspaltprodukte, der Sollwert für den Flächeninhalt einer IR-Absoptionsbande der freigesetzten Abspaltprodukte und/oder der Sollwert für die Intensität einer IR-Absoptionsbande der freigesetzten Abspaltprodukte. Zur Optimierung der Verfahrensdauer wird bevorzugt im Betriebsverfahren beim Erreichen des im Kalibrierverfahren ermittelten Messparameter-Sollwerts oder zu einem festgelegten Zeitpunkt nach Erreichen des Messparameter-Sollwerts die thermische Behandlung beendet.

Der Messparameter-Sollwert wird vorzugsweise im Kalibrierverfahren durch kontinuierlichen oder in festgelegten Zeitabständen stattfindenden Abgleich der Ist-Werte des Performance-Parameters des Testbauteils mit den Soll-Werten des Performance-Parameters des Testbauteils und Bestimmung des zu den jeweiligen Zeitpunkten vorliegenden Messparameter-Istwerts ermittelt.

Weiter bevorzugt ist der Performance-Parameter ein Parameter, der mit dem Vernetzungsgrad des Testbauteils, den mechanischen Eigenschaften des Bauteils, mit dem Restgehalt flüchtiger Bestandteile im Elastomer-Bauteil und/oder dem Restgehalt extrahierbarer Bestandteile korreliert.

Vorzugsweise umfasst das Kalibrierverfahren einen ersten Schritt, in dem mindestens ein vorvernetztes Elastomer-Testbauteil bei einer vorbestimmten Temperatur thermisch behandelt wird, wodurch flüchtige Abspaltprodukte aus dem mindestens einen Elastomer-Testbauteil freigesetzt werden.

Dabei ist der Begriff "vorbestimmte Temperatur" nicht auf eine während des Verfahrens gleichbleibende Temperatur beschränkt. Vielmehr umfasst der Begriff "vorbestimmte Temperatur" auch Temperaturverläufe. In einer bevorzugten Ausführungsform wird das Elastomer-Testbauteil bei einem vorbestimmten Temperaturverlauf thermisch behandelt. Dabei umfasst der Temperaturverlauf vorzugsweise eine Aufheiz-, eine Halte-, und eine Abkühlphase.

In einer Ausführungsform liegt die vorbestimmte Temperatur und/oder zumindest die Temperatur der Haltephase im Bereich von 130 °C bis 220 °C. Für Elastomere ausgewählt aus der Gruppe bestehend aus Siloxanen, vorzugsweise Polysiloxanen, noch bevorzugter Silikonen, insbesondere Liquid Silicon Rubber (LSR) liegt die vorbestimmte Temperatur und/oder zumindest die Temperatur der Haltephase vorzugsweise im Bereich von 200 bis 220 °C. Für Elastomere ausgewählt aus der Gruppe bestehend aus Fluorkautschuk (FKM) und/oder Polyacrylatkautschuk (ACM), liegt die vorbestimmte Temperatur und/oder zumindest die Temperatur der Haltephase vorzugsweise im Bereich von 130 bis 200 °C.

Vorzugsweise wird die thermische Behandlung für einen Zeitraum von mindestens 3 Stunden, beispielsweise 3 Stunden bis 24 Stunden, vorzugsweise 3 Stunden bis 6 Stunden, insbesondere 3,5 bis 4,5 Stunden durchgeführt. Wird ein Elastomer-Testbauteil, das Silikon als Hauptelastomer (mehr als 50 Gew.% bezogen auf die Gesamtmenge an elastomeren Material) aufweist, behandelt, wird die thermische Behandlung vorzugsweise für einen Zeitraum von 0,5 bis 4 h, durchgeführt. Wird ein Elastomer-Testbauteil aus einem elastomeren Material ausgewählt aus der Gruppe FKM, ACM, deren Copolymeren und/oder Blends hiervon als Hauptelastomer (mehr als 50 Gew.% bezogen auf die Gesamtmenge an elastomeren Material) behandelt, wird die thermische Behandlung vorzugsweise für einen Zeitraum von 0,5 bis 24 h durchgeführt.

Die thermische Behandlung wird vorzugsweise in einem Reaktor, insbesondere in einem Ofen durchgeführt, da hierdurch eine einfache Bestimmung der Konzentration der freigesetzten Abspaltprodukte erfolgen kann.

Ebenfalls wird die thermische Behandlung vorzugsweise in mehrere Läufe unterteilt durchgeführt, wobei für jeden Lauf vorzugsweise neue Elastomer-Bauteile verwendet werden. Dies ist vorteilhaft, da so Ungenauigkeiten der Messungen aufgrund des Temperaturabfalls beim Öffnen des Ofens zur Herausnahme von Bauteilen vermieden werden können.

Vorzugsweise umfasst das Kalibrierverfahren einen zweiten Schritt, in dem mindestens ein Messparameter-Istwert, der mit der Ist-Konzentration der freigesetzten Abspaltprodukte und/oder mit der Änderung der Ist-Konzentration der freigesetzten Abspaltprodukte korreliert, vorzugsweise die Ist-Konzentration der freigesetzten Abspaltprodukte, die Änderung der Ist-Konzentration der freigesetzten Abspaltprodukte, der Ist-Wert des Flächeninhalts einer IR-Absoptionsbande der freigesetzten Abspaltprodukte und/oder die Ist-Intensität einer IR-Absoptionsbande der freigesetzten Abspaltprodukte kontinuierlich oder in festgelegten Zeitabständen inline und/oder online, vorzugsweise mittels Infrarot-Gasphasen Spektroskopie, bestimmt wird.

Bei dem erfindungsgemäßen Verfahren hat sich in praktischen Versuchen gezeigt, dass sich ein Infrarot-Gasphasen Spektrometer besonders gut zur quantitativen Bestimmung der Abspaltprodukte eignet, da sich gezeigt hat, dass beim Nachheizprozess von Elastomer-Bauteilen Abspaltprodukte freigesetzt werden, die IR-aktiv sind und ausgeprägte Absorptionsbanden zeigen. Mithin ist ein bevorzugter Messparameter, der mit der Konzentration von flüchtigen Abspaltprodukten aus dem Elastomer-Bauteil korreliert, die Konzentration der freigesetzten Abspaltprodukten selbst. Dabei erfolgt die Bestimmung der Ist-Konzentration der freigesetzten Abspaltprodukte vorzugsweise wie folgt: Es werden IR-Spektren, kontinuierlich oder in festgelegten Zeitabständen, aufgenommen, die für die Abspaltprodukte spezifische Absorptionsbanden zeigen. Zur Bestimmung der Konzentration wird vorzugsweise der Flächeninhalt unter einer oder mehrerer Absorptionsbanden und/oder ihre Intensität als Referenz ausgewählt. Insofern ist ein weiterer bevorzugter Messparameter, der mit der Konzentration von flüchtigen Abspaltprodukten aus dem Elastomer-Bauteil korreliert, der Flächeninhalt einer IR-Absoptionsbande der freigesetzten Abspaltprodukte und/oder die Intensität einer IR-Absoptionsbande der freigesetzten Abspaltprodukte. Bevorzugt werden Absorptionsbanden ausgewählt, die in einem Wellenzahlbereich von 400 cm-1 bis 4000 cm-1 liegen. Ebenfalls geeignet als Messparameter ist die Änderung der Konzentration der freigesetzten Abspaltprodukte.

Im erfindungsgemäßen Verfahren wird vorzugsweise die Veränderung dieser Messparameter über die Zeit der thermischen Behandlung bestimmt.

Mithin sind bevorzugte Messparameter, die mit der Konzentration der freigesetzten Abspaltprodukte und/oder mit der Änderung der Konzentration korrelieren, die Konzentration der freigesetzten Abspaltprodukte, die Änderung der Konzentration der freigesetzten Abspaltprodukte, der Flächeninhalt einer IR-Absorptionsbande der freigesetzten Abspaltprodukte und/oder die Intensität einer IR-Absorptionsbande der freigesetzten Abspaltprodukte.

Vorzugsweise findet die Bestimmung des Messparameters ohne Unterbrechung des Verfahrens statt.

Die Bestimmung des Messparameters, insbesondere des Flächeninhalts und/oder der Intensität einer IR-Absoptionsbande der freigesetzten Abspaltprodukte kann direkt am Reaktionsort erfolgen. Ebenfalls denkbar ist aber auch eine Bestimmung außerhalb des Reaktors. Dazu können die Abspaltprodukte zweckmäßigerweise aus dem Reaktor und/oder der Abluftleitung abgesaugt werden, wobei vorzugsweise Pumpen, insbesondere Vakuumpumpen eingesetzt werden bei einem Absaugdruck, der kleiner ist als der Druck im Reaktor. Geeignete Absaugdrücke liegen beispielweise im Bereich von 700 bis 800 mbar. Wird der Messparameter in der Abluftleitung gemessen, ist es vorteilhaft dies vor Zuführung von Fremdluft durch z.B. Zugunterbrecher oder Leitungszusammenführungen zu tun, um eine Verfälschung des Konzentrationsmesswerts zu vermeiden.

In einer besonders bevorzugten Ausführungsform werden die zu untersuchenden Abspaltprodukte aus dem Reaktor abgesaugt, durch eine, im IR-Gasphasen-Spektrometer enthaltene Küvette, insbesondere Glasküvette geleitet und dort vermessen. Dies hat den Vorteil, dass die Küvette ein definiertes Volumen besitzt und durchlässig für IR-Strahlung ist. Ein weiterer Vorteil liegt darin, dass eine Reinigung der Küvette, beispielsweise durch Spülen mit Sauerstoff oder Stickstoff, ohne Unterbrechung der thermischen Behandlung, möglich ist. Dazu kommt, dass durch die Reinigung ein deutlicheres Messsignal erhalten werden kann.

Der Flächeninhalt und/oder die Intensität der ausgewählten IR-Absoptionsbande der freigesetzten Abspaltprodukte korreliert vorteilhafterweise mit der Konzentration von flüchtigen Abspaltprodukten aus dem Elastomer-Bauteil. Folglich kann der Messparameter-Istwert, beispielsweise die Ist-Konzentration der freigesetzten Abspaltprodukte und/oder ihre Änderung aus dem Flächeninhalt und/oder der Intensität der Absorptionsbande zu einem bestimmten Zeitpunkt oder in einem bestimmten Zeitintervall, mit üblichen Methoden, beispielsweise computerassistiert, bestimmt werden.

Weiter bevorzugt umfasst das Kalibrierverfahren einen dritten Schritt, bei dem in festgelegten Zeitabständen jeweils mindestens ein Testbauteil der thermischen Behandlung entnommen wird, der Ist-Wert mindestens eines Performance-Parameters des Testbauteils bestimmt wird, wobei der Performance-Parameter vorzugsweise mit dem Vernetzungsgrad des Testbauteils, mit dem Restgehalt flüchtiger Bestandteile im Elastomer-Bauteil und/oder dem Restgehalt extrahierbarer Bestandteile korreliert, und wobei der Messparameter-Istwert, der zu dem Zeitpunkt gemessen wird, bei dem ein Testbauteil entnommen wird, das mindestens einen Sollwert des Performance-Parameters des Testbauteils aufweist, dem Messparameter-Sollwert entspricht.

Vorzugsweise werden die bestimmten Messparameter-Istwerte graphisch in Form einer Ist-Wertkurve dargestellt.

Wie oben dargelegt, kann die Bestimmung der Messparameter direkt im Reaktor erfolgen. Ebenfalls denkbar ist aber auch eine Bestimmung außerhalb des Reaktors.

Bevorzugte Performance-Parameter sind Parameter des Bauteils, die ab einem bestimmten Zeitpunkt der thermischen Behandlung konstant bleiben.

Bevorzugte Performance-Parameter, die mit dem Vernetzungsgrad des Bauteils korrelieren, sind Zugspannung nach DIN 53504:2017-03, Reißdehnung nach DIN 53504:2017-03, Druckverformungsrest nach DIN ISO 815-1:2022-04 und/oder Shore A Härte nach DIN ISO 7619-1 :2012-02. Bevorzugte Sollwerte für die Zugspannung sind 4 MPa bis 12 MPa, noch bevorzugter 7 MPa bis 12 MPa. Bevorzugte Sollwerte für die Reißdehnung sind 400% bis 750%. Bevorzugte Sollwerte für den Druckverformungsrest sind kleiner als 30%, noch bevorzugter kleiner als 15%. Mithin wählt man für die Durchführung des erfindungsgemäßen Verfahrens vorzugsweise Sollwerte aus, die innerhalb der vorgenannten Bereiche liegen.

Bevorzugte mit dem Restgehalt flüchtiger Bestandteile und/oder extrahierbarer Bestandteile im Elastomer-Bauteil korrelierende Performance-Parameter für Elastomer-Bauteile, die Siloxane enthalten, vorzugsweise Polysiloxane und noch bevorzugter Silikone, insbesondere Liquid Silicon Rubber (LSR), sind der Extraktionswert für flüchtige Bestandteile, insbesondere für Dodecamethylcyclohexasiloxan, Decamethylcyclopentasiloxan, Octamethylcyclotetrasiloxan zusammen genommen, gemessen als Masseverlust (DIN ISO 1817:2015). Bevorzugte Sollwerte für den Extraktionswert flüchtiger Bestandteile, bevorzugt für Dodecamethylcyclohexasiloxan, Decamethylcyclopentasiloxan und Octamethylcyclotetrasiloxan zusammengenommen, sind kleiner als 2 Gew.%, noch bevorzugter kleiner als 0,5 Gew.%.

Bevorzugte Zeitabstände für die Entnahme des Testbauteils sind von 5 bis 10 h, vorzugszuweise von 1 bis 5 h, noch bevorzugter von 0,5 bis 1 h.

Falls das Betriebsverfahren mit anderen Bedingungen als das Kalibrierverfahren durchgeführt werden soll, d.h. z.B. mit Bauteilen anderer Abmessungen oder Formen, mit anderen Ofenbeladungen, Luftwechselraten, Temperaturverläufen und/oder anderen Temperaturen, kann sich an den dritten Schritt der optionale Schritt 4 anschließen, in dem die in Schritt 3 ermittelten Messparameter-Sollwerte an die anderen Bedingungen des Betriebsverfahrens adaptiert werden.

Die Adaption des Messparameter-Sollwerts kann auf dem Fachmann übliche Weise durchgeführt werden. So kann beispielsweise, wenn das Betriebsverfahren mit anderen Ofenbeladungen als im Kalibrierverfahren durchgeführt werden soll, das Kalibrierverfahren einmal mit einer minimalen Ofenbeladung und dann noch einmal mit einer maximalen Ofenbeladung durchgeführt werden und jeweils, wie oben beschrieben, mindestens ein Messparameter-Sollwert bestimmt werden. Dabei werden minimale und maximale Ofenbeladung vorzugsweise so gewählt, dass die für das Betriebsverfahren gewählten Ofenbeladungen zwischen minimaler und maximaler Ofenbeladung liegen. Anschließend kann der Messparameter-Sollwert auf weitere Ofenbeladungen interpoliert werden.

Soll das Betriebsverfahren mit anderen Luftwechselraten als im Kalibrationsverfahren durchgeführt werden, kann eine Adaption des Messparameter-Sollwerts analog zur Adaption im Hinblick auf eine geänderte Ofenbeladung über Interpolation des ermittelten Messparameter-Sollwerts mit minimaler und maximaler Luftwechselrate erfolgen. Wird die Luftwechselrate im Betriebsverfahren dynamisch angepasst, das heißt herrschen während eines Prozesszyklus verschiedene Luftwechselraten, so kann über einen Strömungsmesser im Abluftsystem das den Reaktor durchströmte Luftvolumen bestimmt werden und der Messparameter-Sollwert damit adaptiert werden. Damit ist eine Vergleichbarkeit der Messparameter-Sollwerte bei beliebiger Luftwechselrate hergestellt.

In einer weiteren bevorzugten Ausführungsform umfasst das erfindungsgemäße Verfahren ein Betriebsverfahren B.

Das Betriebsverfahren umfasst vorzugsweise einen Schritt 1', in dem mindestens ein vorvernetztes Elastomer-Testbauteil bei einer vorbestimmten Temperatur thermisch behandelt wird, wodurch flüchtige Abspaltprodukte aus dem mindestens einen Testbauteil freigesetzt werden.

Die thermische Behandlung erfolgt vorzugsweise in einem Reaktor, insbesondere in einem Ofen, da hierdurch eine einfache Bestimmung der Konzentration der freigesetzten Abspaltprodukte erfolgen kann.

In einer Ausführungsform entsprechen die Bedingungen des Betriebsverfahrens denen des Kalibrierverfahrens, da hierdurch eine Adaption des im Kalibrierverfahren ermittelten Messparameter-Sollwerts vermieden werden kann. Zu diesem Zweck entspricht vorzugsweise die Temperatur des Betriebsverfahrens der des Kalibrierverfahrens. Ferner werden im Betriebsverfahren in einer Ausführungsform Elastomer-Bauteile verwendet, die baugleich zu dem mindestens einem Testbauteil sind. Unter baugleich ist dabei zu verstehen, dass Testbauteil und Bauteil aus den gleichen Materialien bestehen, die gleichen Abmessungen und das gleiche Oberflächen-/Volumenverhältnis besitzen.

Das Betriebsverfahren umfasst weiter bevorzugt einen Schritt 2', in dem der Messparameter-Istwert kontinuierlich oder in festgelegten Zeitabständen inline und/oder online, vorzugsweise mittels Infrarot-Gasphasen Spektroskopie, bestimmt wird. Bevorzugte Ausführungsformen dieses Verfahrensschritts umfassen die in Bezug auf Schritt 2) des Kalibrierverfahrens beschriebenen Ausführungsformen mutatis mutandis.

Weiter bevorzugt umfasst das Betriebsverfahren einen Schritt 3', in dem der Messparameter-Istwert mit dem im Kalibrierverfahren ermittelten Messparameter-Sollwert verglichen und dieser Abgleich zur Einstellung von Verfahrensparametern des Betriebsverfahrens, beispielsweise der Dauer der thermischen Behandlung, der Luftwechselrate und/oder des Temperaturverlaufs verwendet wird. Der Vergleich wird vorzugsweise automatisiert und/oder computerassistiert ausgeführt. Noch bevorzugter wird der Vergleich mit Hilfe einer Auswertungssoftware ausgeführt, die beispielsweise in einem IR-Spektrometer integriert ist.

In einer bevorzugten Ausführungsform besteht die Einstellung von Verfahrensparametern des Betriebsverfahrens darin, dass die Temperaturbehandlung des mindestens einen Elastomer-Bauteils, vorzugsweise automatisiert, beendet und/oder die Temperaturbehandlung für einen bestimmten festgelegten Zeitraum weitergeführt und die Temperaturbehandlung, vorzugsweise automatisiert, beendet wird, sobald der Messparameter-Istwert dem Messparameter-Sollwert entspricht oder diesen unter- oder überschreitet, vorzugsweise sobald der Istwert des Flächeninhalts einer IR-Absoptionsbande und/oder die Intensität einer IR-Absoptionsbande ihren/seinen Sollwert unterschreitet. Bevorzugt findet die Beendigung und/oder Fortführung der Temperaturbehandlung für einen bestimmten festgelegten Zeitraum bei Unterschreitung des Messparameter-Sollwerts statt. Der Grund dafür ist, dass viele Messparameter während der thermischen Behandlung zunächst ansteigen und anschließend wieder abfallen und somit eine Kurve bilden. Es hat sich gezeigt, dass der abfallende Bereich dieser Kurve besonders geeignet zur Festlegung des Messparameter-Sollwerts ist.

Die Beendigung der Temperaturbehandlung erfolgt vorzugsweise computergesteuert, noch bevorzugter durch einen Programbefehl der Auswertungssoftware, die beispielsweise in einem IR-Spektrometer integriert ist.

Ein weiterer Gegenstand der Erfindung ist ein Verfahren zur thermischen Behandlung eines vorvernetzten Elastomer-Bauteils, umfassend folgende Schritte:
**A) mindestens ein Kalibrierverfahren, umfassend:**
   Schritt 1: mindestens ein vorvernetztes Elastomer-Testbauteil wird bei einer vorbestimmten Temperatur thermisch behandelt, wodurch flüchtige Abspaltprodukte aus dem mindestens einen Testbauteil freigesetzt werden;
   Schritt 2: mindestens ein Messparameter-Istwert, der mit der Ist-Konzentration der freigesetzten Abspaltprodukte und/oder mit der Änderung der Ist-Konzentration der freigesetzten Abspaltprodukte korreliert, vorzugsweise die Ist-Konzentration der freigesetzten Abspaltprodukte, die Änderung der Ist-Konzentration der freigesetzten Abspaltprodukte, der Ist-Wert des Flächeninhalts einer IR-Absoptionsbande der freigesetzten Abspaltprodukte und/oder der Ist-Wert der Intensität einer IR-Absoptionsbande der freigesetzten Abspaltprodukte, wird kontinuierlich oder in festgelegten Zeitabständen inline und/oder online, vorzugsweise mittels Infrarot-Gasphasen Spektroskopie, bestimmt;
   Schritt 3: in festgelegten Zeitabständen wird jeweils mindestens ein Testbauteil der thermischen Behandlung entnommen und es wird der Ist-Wert mindestens eines Performance-Parameters des Testbauteils bestimmt, wobei der Performance-Parameter vorzugsweise mit dem Vernetzungsgrad des Testbauteils, mit dem Restgehalt flüchtiger Bestandteile im Elastomer-Bauteil und/oder dem Restgehalt extrahierbarer Bestandteile korreliert; und wobei der Messparameter-Istwert, der zu dem Zeitpunkt gemessen wird, bei dem ein Testbauteil entnommen wird, das mindestens einen Sollwert des Performance-Parameters des Testbauteils aufweist, dem Messparameter-Sollwert entspricht;
   Schritt 4: falls das Betriebsverfahren mit anderen Bedingungen als das Kalibrierverfahren durchführt werden soll, vorzugsweise mit anderen Ofenbeladungen, Luftwechselraten, Temperaturverläufen und/oder anderen Temperaturen, wird der in Schritt 3 erhaltene Messparameter-Sollwert gegebenenfalls an die anderen Bedingungen adaptiert;
**B)mindestens ein Betriebsverfahren, umfassend:**
   Schritt 1': mindestens ein vorvernetztes Elastomer-Bauteil wird bei einer vorbestimmten Temperatur thermisch behandelt, wodurch flüchtige Abspaltprodukte aus dem Bauteil freigesetzt werden;
   Schritt 2': der Messparameter-Istwert wird kontinuierlich oder in festgelegten Zeitabständen inline und/oder online mittels Infrarot-Gasphasen Spektroskopie bestimmt;
   Schritt 3': der Messparameter-Istwert wird mit dem im Kalibrierverfahren ermittelten Messparameter-Sollwert, verglichen und dieser Abgleich wird zur Einstellung von Verfahrensparametern des Betriebsverfahrens verwendet.

Bevorzugte Ausführungsformen des Verfahrens umfassen die erfindungsgemäß beschriebenen.

Das erfindungsgemäße Verfahren kann auch zur Optimierung der Luftwechselrate und/oder des Temperaturverlaufs verwendet werden. So ist ein bevorzugter anhand der Konzentrationsänderung von flüchtigen Abspaltprodukten im erfindungsgemäßen Verfahren einstellbarer Verfahrensparameter die Luftwechselrate und/oder der Temperaturverlauf.

Hierzu kann in einem ersten Schritt im Kalibrierverfahren a) eine Grenzkonzentration der Abspaltprodukte ermittelt werden, deren Überschreiten einen unerwünschten Einfluss auf das Verfahren hat, beispielsweise zur Ausbildung einer entzündbaren Atmosphäre, insbesondere im Reaktor, zu einer zu hohen Schadstoffkonzentration im Reaktor und/oder zu einer Überschreitung von Emissionsgrenzwerten führt, und/oder b) ein Messparametergrenzwert ermittelt werden, der mit der Grenzkonzentration der Abspaltprodukte korreliert und dessen Überschreiten einen unerwünschten Einfluss auf das Verfahren hat, beispielsweise zur Ausbildung einer entzündbaren Atmosphäre, insbesondere im Reaktor, zu einer zu hohen Schadstoffkonzentration im Reaktor und/oder zu einer Überschreitung von Emissionsgrenzwerten, führt, und/oder c) eine Maximalkonzentration der Abspaltprodukte ermittelt werden, die in einem festgelegten Abstand unterhalb ihrer Grenzkonzentration liegt und/oder d) ein Messparametermaximalwert ermittelt werden, der in einem festgelegten Abstand unterhalb des Messparametergrenzwerts liegt.

Dabei ist der Messparametergrenzwert und/oder der Messparametermaximalwert vorzugsweise ausgewählt aus Grenzwerten und/oder Maximalwerten für die Konzentration der freigesetzten Abspaltprodukte, die Änderung der Konzentration der freigesetzten Abspaltprodukte, den Flächeninhalt einer IR-Absoptionsbande der freigesetzten Abspaltprodukte und/oder den Ist-Wert der Intensität einer IR-Absoptionsbande der freigesetzten Abspaltprodukte.

Im Betriebsverfahren können dann die Ist-Konzentrationen der Abspaltprodukte kontinuierlich oder in festgelegten Zeitabständen mit ihrer im Kalibrierverfahren ermittelten Grenzkonzentration und/oder ihrer Maximalkonzentration verglichen und die Luftwechselrate unter Berücksichtigung der Grenzkonzentration oder der Maximalkonzentration eingestellt werden.

Analog können im Betriebsverfahren die Messparameter-Istwerte kontinuierlich oder in festgelegten Zeitabständen mit ihrem im Kalibrierverfahren ermittelten Messparametergrenzwert oder ihrem Messparametermaximalwert verglichen und die Luftwechselrate unter Berücksichtigung der Grenzkonzentration, der Maximalkonzentration, des Messparametergrenzwerts und/oder des Messparametermaximalwerts eingestellt werden.

In einer besonders bevorzugten Ausführungsform wird dabei die Luftwechselrate nur so hoch eingestellt, dass die Ist-Konzentration der Abspaltprodukte noch unterhalb der Grenzkonzentration oder der Maximalkonzentration liegt. Zu diesem Zweck kann die Luftwechselrate erhöht werden, wenn die Ist-Konzentrationen der Abspaltprodukte ihre im Kalibrierverfahren ermittelte Grenzkonzentration oder Maximalkonzentration überschreitet und/oder wenn der Messparameter-Istwert seinen im Kalibrierverfahren ermittelten Messparametergrenzwert und/oder Messparametermaximalwert überschreitet. Ferner wird die Luftwechselrate bevorzugt so eingestellt, dass die Ist-Konzentration der Abspaltprodukte unterhalb der Grenzkonzentration oder der Maximalkonzentration liegt und/oder der Messparameter-Istwert unterhalb des Messparametergrenzwerts und/oder Messparametermaximalwerts liegt aber die Luftwechselrate dennoch möglichst niedrig ist.

In einer weiteren bevorzugten Ausführungsform wird im Kalibrierverfahren anhand der Konzentrationsänderung der flüchtigen Abspaltprodukte und/oder anhand der Änderung des Messparameter-Istwerts eine minimale Luftwechselrate und/oder ein optimierter Luftwechselratenverlauf ermittelt und im Betriebsverfahren diese minimale Luftwechselrate und/oder der optimierter Luftwechselratenverlauf eingestellt.

Ein weiterer Gegenstand der Erfindung umfasst ein vernetztes Elastomer-Bauteil, das mit dem erfindungsgemäßen Verfahren thermisch behandelt wurde.

In einer bevorzugten Ausführungsform weist das vernetzte Elastomer-Bauteil einen oder mehrere der folgenden Performance-Parameter auf:
- eine Zugspannung gemessen nach DIN 53504:2017-03 von 4 MPa bis 12 MPa, noch bevorzugter von 7 MPa bis 12 MPa;
- eine Reißdehnung gemessen nach DIN 53504:2017-03 von 400% bis 750%;
- einen Druckverformungsrest gemessen nach DIN ISO 815-1:2022-04 von kleiner 30%, noch bevorzugter kleiner 15%;
- einen Extraktionswert von <2,0 Gew. % flüchtige Bestandteile, gemessen als Masseverlust (DIN ISO 1817:2015) und/oder
- einen Anteil an flüchtigen Bestandteilen < 100 ppm.

Vorteilhaft an einem Extraktionswert von <2,0 Gew. % flüchtige Bestandteile, gemessen als Masseverlust (DIN ISO 1817:2015) und/oder einem Anteil an flüchtigen Bestandteilen, von < 100 ppm ist beispielsweise, dass die hohen an Medizinprodukte gestellten Reinheitsanforderungen (vgl. Pharmacopoeia) erfüllt werden können.

Im Folgenden wird die Erfindung anhand mehrerer nicht beschränkender Beispiele näher erläutert.

### Beispiel 1:

### A) Kalibrierverfahren

### Schritt 1:

100 Stück aus Flüssigsilikonkautschuk bestehende, durch Spritzgussverfahren in Form gebrachte (in Form eines Ringes), somit vorvernetzte, Elastomer-Testbauteile werden nach dem Auswurf aus der Kavität, händisch einem Nachheizofen zugeführt.

Hierfür werden diese vorvernetzten Bauteile unter Verwendung eines Trägers im Nachheizofen platziert (Elastomer-Bauteile werden auf einem Gitter platziert). Es kann auch eine siebförmige Trommel mit Elastomer-Bauteilen bestückt und diese dann im Ofen platziert werden.

Der Nachheizofen wird geschlossen. Das Heizprogramm des Ofens wird gestartet, wodurch der Nachheiz-Prozess und somit die thermische Behandlung der Bauteile beginnt. Der Nachheizprozess unterteilt sich in drei Phasen: der Aufheiz-, der Halte-, und der Abkühlphase.

Nach einer Aufheizphase von 20 Minuten erreicht der Ofen die Temperatur der Haltephase von 200°C. Diese Temperatur wird für 4,5 Stunden gehalten. Nach Ablauf dieser Zeit beginnt die Abkühlphase, die 20 min dauert und in welcher der Ofen keine Energiezufuhr erfährt.

Während des Nachheizprozesses kommt es zu chemischen Vorgängen (z. B. Umlagerungsreaktionen) innerhalb der Bauteile. Dabei werden als flüchtige Abspaltprodukte Dodecamethylcyclohexasiloxan (D6), Decamethylcyclopentasiloxan (D5) und/oder Octamethylcyclotetrasiloxan (D4) freigesetzt.

### Schritt 2:

Durch Nutzung einer Vakuumpumpe werden die freigesetzten, flüchtigen Abspaltprodukte aus der dem Reaktorraum nachgeschalteten Absaugleitung abgesaugt und der Messküvette eines Gasphasen-IR-Spektrometers zugeführt. In Zeitabständen von 2 min wird das Messsignal des Spektrometers an einen Computer übermittelt. Hierdurch wird als Messparameter-Istwert die Intensität einer IR-Absorptionsbande der freigesetzten Abspaltprodukte bestimmt.

### Schritt 3:

Die thermische Behandlung wird in mehrere Läufe unterteilt durchgeführt, wobei für jeden Lauf neue Bauteile verwendet werden. Dabei wird darauf geachtet, dass die Ofenbeladung (Anzahl und Art der Bauteile) und die Ofenparameter (Zeit und Temperatur, Luftwechselrate) zwischen den Läufen identisch ist. Der Grund für die Durchführung mehrerer Läufe ist, dass bei der Probeentnahme der Ofen und das Testbauteil abkühlt und sich die Konzentration der Abspaltprodukte ändert.

Im Lauf Nummer 1 werden nach insgesamt 20 min 10 Testbauteile der thermischen Behandlung (Ende Aufheizphase) entnommen und die folgenden Performance-Parameter bestimmt:
Zugspannung (DIN 53504:2017-03)
Reißdehnung (DIN 53504:2017-03)
Druckverformungsrest (DIN ISO 815-1:2022-04)
Shore A Härte (DIN ISO 7619-1:2012-02)

Extraktionswerte für flüchtige Bestandteile, insbesondere für Dodecamethylcyclohexasiloxan, Decamethylcyclopentasiloxan, Octamethylcyclotetrasiloxan zusammengenommen, mit n-Hexan im Ultraschall (1 Std. 60 °C) und anschließender GC/MS-Analyse des Extrakts Extraktionswerte für flüchtige Bestandteile, insbesondere für Dodecamethylcyclohexasiloxan, Decamethylcyclopentasiloxan, Octamethylcyclotetrasiloxan zusammen genommen, mit n-Hexan und anschließender GC/MS-Analyse des Extrakts (Soxhlet), gemessen als Masseverlust (DIN ISO 1817:2015).

Im Lauf Nummer 2 werden nach insgesamt 40 min 10 Testbauteile der thermischen Behandlung (entspricht z. B. einer 20-minütigen Nachheizzeit bei 200°C) entnommen und wiederum die oben genannten Performance-Parameter bestimmt.

Im Anschluss werden analog weitere Läufe durchgeführt bis zum Ende der Abkühlphase.

Im Anschluss erfolgt die Auswertung zur Bestimmung eines Messparameter-Sollwerts der Abspaltprodukte. Hierzu wird geprüft bei welchem Messparameter-Istwert der freigesetzten Abspaltprodukte, ein Testbauteil entnommen wurde, das mindestens einen Sollwert des Performance-Parameters des Testbauteils entspricht. Im vorliegenden Fall wird als Performance-Parameter des Testbauteils der Extraktionswert für Dodecamethylcyclohexasiloxan, Decamethylcyclopentasiloxan, Octamethylcyclotetrasiloxan zusammen genommen, gemessen als Masseverlust (DIN ISO 1817:2015 gewählt. Dessen Sollwert von kleiner als 2 Gew.% wurde nach 4 Stunden erreicht. Zu diesem Zeitpunkt wurde ein IR-Signal mit einer Fläche von 0,38 gemessen. Diese Fläche korreliert mit der Soll-Konzentration der Abspaltprodukte und wird für das nachfolgende Betriebsverfahren als Messparameter-Sollwert festgesetzt.

### B) Betriebsverfahren

### Schritt 1':

Aus Flüssigsilikonkautschuk bestehende, durch Spritzgussverfahren in Form gebrachte (in Form von Ringen), somit vorvernetzte, Elastomerbauteile werden nach dem Auswurf aus der Kavität, händisch einem Nachheizofen zugeführt.

Hierfür werden diese vorvernetzten Bauteile unter Verwendung eines Trägers im Nachheizofen platziert (Elastomer-Bauteile werden auf einem Gitter platziert). Es kann auch eine siebförmige Trommel mit Elastomer-Bauteilen bestückt und diese dann im Ofen platziert werden.

Der Nachheizofen wird geschlossen. Das Heizprogramm des Ofens wird gestartet, wodurch der Nachheiz-Prozess und somit die thermische Behandlung der Bauteile beginnt. Der Nachheizprozess unterteilt sich in drei Phasen. Der Aufheiz-, der Halte-, und der Abkühlphase.

Nach einer Aufheizphase von 20 Minuten erreicht der Ofen die Temperatur der Haltephase von 200°C.

### Schritt 2':

Durch Nutzung einer Vakuumpumpe werden die freigesetzten, flüchtigen Abspaltprodukte aus der dem Reaktorraum nachgeschalteten Absaugleitung abgesaugt und der Messküvette eines Gasphasen-IR-Spektrometers zugeführt. In Zeitabständen von 2 min wird das Messsignal des Spektrometers an einen Computer übermittelt. Hierdurch wird als Messparameter-Istwert die Intensität einer IR-Absorptionsbande der freigesetzten Abspaltprodukte bestimmt.

### Schritt 3':

Der Messparameter-Istwert, hier der Ist-Wert der Intensität einer IR-Absorptionsbande, wird mit dem im Kalibrierverfahren ermittelten Messparameter-Sollwert, d.h. dem Soll-Wert der Intensität der IR-Absorptionsbande verglichen.

Die Temperaturbehandlung wird automatisiert beendet, sobald die Fläche der IR-Absorptionsbande ihren Messparameter-Sollwert, d.h. den Sollwert der Fläche der IR-Absorptionsbande (Intensität von 0,38) unterschreitet.

### Beispiel 2:

### A) Kalibrierverfahren

### Schritt 1:

100 Stück aus Polyacrylkautschuk (ACM) bestehende, durch Spritzgussverfahren in Form gebrachte (in Form eines Radialwellendichtrings), somit vorvernetzte, Elastomer-Testbauteile werden nach dem Auswurf aus der Kavität, händisch einem Nachheizofen zugeführt.

Hierfür werden diese vorvernetzten Bauteile unter Verwendung eines Trägers im Nachheizofen platziert (Elastomer-Bauteile werden auf einem Gitter platziert). Es kann auch eine siebförmige Trommel mit Elastomer-Bauteilen bestückt und diese dann im Ofen platziert werden.

Der Nachheizofen wird geschlossen. Das Heizprogramm des Ofens wird gestartet, wodurch der Nachheiz-Prozess und somit die thermische Behandlung der Bauteile beginnt. Der Nachheizprozess unterteilt sich in drei Phasen: der Aufheiz-, der Halte-, und der Abkühlphase.

Während des Nachheizprozesses kommt es zu chemischen Vorgängen (z. B. Umlagerungsreaktionen) innerhalb der Bauteile. Dabei werden als flüchtige Abspaltprodukte Kohlenstoffmonoxid, Schwefeldioxid, Stickstoffdioxid, Kohlenstoffdioxid freigesetzt.

Es werden insgesmat 3 Kalibrierverfahren durchgeführt. Es wurden dabei folgende Luftwechselraten verwendet. 50 m³/Std (1. Kalibrierverfahren), 100 m³/Std (2. Kalibrierverfahren) und 200 m³/Std (3. Kalibrierverfahren).

### Schritt 2:

Durch Nutzung einer Vakuumpumpe werden die freigesetzten, flüchtigen Abspaltprodukte aus der dem Reaktorraum nachgeschalteten Absaugleitung abgesaugt und einem Flammenionisations-Detektor zugeführt. In Zeitabständen von 15 Sekunden wird das Messsignal des Flammenionisations-Detektors an einen Computer übermittelt.

### Schritt 3:

Die thermische Behandlung wird in mehrere Läufe unterteilt durchgeführt, wobei für jeden Lauf neue Bauteile verwendet werden. Dabei wird darauf geachtet, dass die Ofenbeladung (Anzahl und Art der Bauteile) und die Ofenparameter (Zeit und Temperatur, Luftwechselrate) zwischen den Läufen identisch ist. Der Grund für die Durchführung mehrerer Läufe ist, dass bei der Probeentnahme der Ofen und das Testbauteil abkühlt und sich die Konzentration der Abspaltprodukte ändert.

Im Lauf Nummer 1 werden 10 Testbauteile der thermischen Behandlung (Ende Aufheizphase) entnommen und die folgenden Performance-Parameter bestimmt:
Zugspannung (DIN 53504:2017-03)
Reißdehnung (DIN 53504:2017-03)
Druckverformungsrest (DIN ISO 815-1:2022-04)
IRHD (DIN ISO 48:2021-02)
Dichte
Masseverlust (DIN ISO 1817:2015).

Im Lauf Nummer 2 werden 10 Testbauteile der thermischen Behandlung entnommen und wiederum die oben genannten Performance-Parameter bestimmt.

Im Anschluss werden analog weitere Läufe durchgeführt bis zum Ende der Abkühlphase.

Im Anschluss erfolgt die Auswertung zur Bestimmung eines Messparameter-Sollwerts der Abspaltprodukte. Hierzu wird geprüft bei welchem Messparameter-Istwert der freigesetzten Abspaltprodukte, ein Testbauteil entnommen wurde, das mindestens einen Sollwert des Performance-Parameters des Testbauteils entspricht. Im vorliegenden Fall wird als Performance-Parameter Zugspannung (DIN 53504:2017-03),
Reißdehnung (DIN 53504:2017-03),
Druckverformungsrest (DIN ISO 815-1:2022-04),
IRHD (DIN ISO 48:2021-02),
Dichte,
Masseverlust (DIN ISO 1817:2015) bestimmt.

Es wurde gefunden, dass bei sämtlichen Luftwechselraten die Testbauteile die Performance-Parameter aufwiesen nach Abschluss des Kalibrierverfahrens. Es wurde ferner gefunden das bei einer Luftwechselrate von 50 m³/Std der Grenzwert für VOC von 1500 mg-C/m³ überschritten wurde, bei den beiden höheren Luftwechselraten jedoch nicht. Für das Betriebsverfahren wurde die Luftwechselrate von 100 m³/Std ausgewählt, da sie aus energetischen Gründen gegenüber der 200 m³/Std bevorzugt ist.

### B) Betriebsverfahren

### Schritt 1:

100 Stück aus Polyacrylkautschuk (ACM) bestehende, durch Spritzgussverfahren in Form gebrachte (in Form eines Radialwellendichtrings), somit vorvernetzte, Elastomer-Testbauteile werden nach dem Auswurf aus der Kavität, händisch einem Nachheizofen zugeführt.

Hierfür werden diese vorvernetzten Bauteile unter Verwendung eines Trägers im Nachheizofen platziert (Elastomer-Bauteile werden auf einem Gitter platziert). Es kann auch eine siebförmige Trommel mit Elastomer-Bauteilen bestückt und diese dann im Ofen platziert werden.

Der Nachheizofen wird geschlossen. Das Heizprogramm des Ofens wird gestartet, wodurch der Nachheiz-Prozess und somit die thermische Behandlung der Bauteile beginnt. Der Nachheizprozess unterteilt sich in drei Phasen: der Aufheiz-, der Halte-, und der Abkühlphase.

Während des Nachheizprozesses kommt es zu chemischen Vorgängen (z. B. Umlagerungsreaktionen) innerhalb der Bauteile. Dabei werden als flüchtige Abspaltprodukte Kohlenstoffmonoxid, Schwefeldioxid, Stickstoffdioxid, Kohlenstoffdioxid freigesetzt.

Es wird eine Luftwechselrate von 100 m³/Std verwendet.

## Patentansprüche

1. Verfahren zur thermischen Behandlung mindestens eines vorvernetzten Elastomer-Bauteils, **dadurch gekennzeichnet, dass** die Änderung mindestens eines Messparameters, der mit der Konzentration von flüchtigen Abspaltprodukten und/oder mit der Änderung der Konzentration von flüchtigen Abspaltprodukten aus dem Elastomer-Bauteil korreliert, während der thermischen Behandlung gemessen wird und die erhaltenen Messwerte zur Einstellung von mindestens einem Verfahrensparameter verwendet werden.

2. Verfahren nach Anspruch 1, **dadurch gekennzeichnet, dass** der mindestens eine Messparameter ausgewählt ist aus der Konzentration der freigesetzten Abspaltprodukte, der Änderung der Konzentration der freigesetzten Abspaltprodukte, dem Flächeninhalt einer IR-Absoptionsbande der freigesetzten Abspaltprodukte und/oder der Intensität einer IR-Absoptionsbande der freigesetzten Abspaltprodukte.

3. Verfahren nach Anspruch 1 oder 2, **dadurch gekennzeichnet, dass** die Messung der Änderung des Messparameters inline und/oder online, insbesondere ohne Unterbrechung des Verfahrens stattfindet.

4. Verfahren nach einem oder mehreren der vorangehenden Ansprüche,
**dadurch gekennzeichnet, dass** der mindestens eine Verfahrensparameter die Dauer der thermischen Behandlung, die Luftwechselrate und/oder den Temperaturverlauf, insbesondere die Luftwechselrate und/oder den Temperaturverlauf, umfasst.

5. Verfahren nach einem oder mehreren der vorangehenden Ansprüche,
**dadurch gekennzeichnet, dass** die erhaltenen Messwerte wie folgt zur Einstellung des mindestens einen Verfahrensparameters verwendet werden:
A1) die erhaltenen Messwerte werden in mindestens einem Kalibrierverfahren zur Ermittlung einer Grenzkonzentration der flüchtigen Abspaltprodukte verwendet, deren Überschreiten einen unerwünschten Einfluss auf das Verfahren hat, und
A2) in mindestens einem Betriebsverfahren wird die Luftwechselrate erhöht und/oder dieTemperatur verringert, sobald die Konzentration der flüchtigen Abspaltprodukten einen Wert erreicht, der in einem festgelegten Abstand von der Grenzkonzentration liegt.

6. Verfahren nach einem oder mehreren der vorangehenden Ansprüche, **dadurch gekennzeichnet, dass** die erhaltenen Messwerte wie folgt zur Einstellung des mindestens einen Verfahrensparameters verwendet werden:
B1) die erhaltenen Messwerte werden in mindestens einem Kalibrierverfahren zur Ermittlung eines optimierten Luftwechselratenverlaufs verwendet und
B2) in mindestens einem Betriebsverfahren wird der optimierte Luftwechselratenverlauf eingestellt
und/oder
C1) die erhaltenen Messwerte werden in mindestens einem Kalibrierverfahren zur Ermittlung eines optimierten Temperaturverlaufs verwendet und
C2) der optimierte Temperaturverlauf wird in mindestens einem Betriebsverfahren eingestellt.

7. Verfahren nach einem oder mehreren der vorangehenden Ansprüche,
**dadurch gekennzeichnet, dass** die flüchtigen Abspaltprodukte so ausgewählt werden, dass ihre Konzentration einen Rückschluss auf einen Performance-Parameter des Elastomer-Bauteils ermöglicht, vorzugsweise auf den Umsetzungsgrad der Nachvernetzungsreaktion des Elastomer-Bauteils, auf den Restgehalt flüchtiger Bestandteile im Elastomer-Bauteil und/oder auf den Restgehalt extrahierbarer Bestandteile im Elastomer-Bauteil, insbesondere auf den Umsetzungsgrad der Nachvernetzungsreaktion des Elastomer-Bauteils und/oder die mechanischen Eigenschaften des Bauteils.

8. Verfahren nach einem oder mehreren der vorangehenden Ansprüche,
**dadurch gekennzeichnet, dass** das Verfahren ein Kalibrierverfahren und ein Betriebsverfahren umfasst.

9. Verfahren nach Anspruch 5 bis 6 oder 8, **dadurch gekennzeichnet, dass** mit dem Kalibrierverfahren ein Messparameter-Sollwert festgelegt wird, der mit dem Soll-Wert des Performance-Parameters korreliert.

10. Verfahren nach Anspruch 9, **dadurch gekennzeichnet, dass** der Messparameter-Sollwert durch kontinuierlichen oder in festgelegten Zeitabständen stattfindenden Abgleich der Ist-Werte des Performance-Parameters eines Elastomer-Testbauteils mit den Soll-Werten des Performance-Parameters des Elastomer-Testbauteils sowie dem hierzu korrelierenden Messparameter-Istwert der Abspaltprodukte ermittelt wird.

11. Verfahren nach einem oder mehreren der Ansprüche 8 bis 10, **dadurch gekennzeichnet, dass** mit dem Betriebsverfahren eine thermische Behandlung des vorvernetzten Elastomer-Bauteils durchgeführt wird, wobei während der thermischen Behandlung ein Vergleich der Messparameter-Istwerte mit dem im Kalibrierverfahren ermittelten Messparameter-Sollwert erfolgt und zur Einstellung von Verfahrensparametern verwendet wird.

12. Verfahren nach einem oder mehreren der Ansprüche 8 bis 11, **dadurch gekennzeichnet, dass** im Betriebsverfahren beim Erreichen des im Kalibrierverfahren ermittelten Messparameter-Sollwerts, vorzugsweise beim Erreichen des Sollwerts für den Flächeninhalt einer IR-Absoptionsbande der freigesetzten Abspaltprodukte, oder zu einem festgelegten Zeitpunkt nach Erreichen des Messparameter-Sollwerts die thermische Behandlung beendet wird.

13. Verfahren nach einem oder mehreren der Ansprüche 8 bis 12, **dadurch gekennzeichnet, dass** im Kalibrierverfahren a) eine Grenzkonzentration der Abspaltprodukte ermittelt wird, deren Überschreiten einen unerwünschten Einfluss auf das Verfahren hat, beispielsweise zur Ausbildung einer entzündbaren Atmosphäre, insbesondere im Reaktor, führt, und/oder b) ein Messparametergrenzwert ermittelt wird, der mit der Grenzkonzentration der Abspaltprodukte korreliert und dessen Überschreiten einen unerwünschten Einfluss auf das Verfahren hat, beispielsweise zur Ausbildung einer entzündbaren Atmosphäre, insbesondere im Reaktor, führt, und/oder c) eine Maximalkonzentration der Abspaltprodukte ermittelt wird, die in einem festgelegten Abstand unterhalb ihrer Grenzkonzentration liegt und/oder d) ein Messparametermaximalwert ermittelt werden, der in einem festgelegten Abstand unterhalb des Messparametergrenzwerts liegt.

14. Verfahren nach einem oder mehreren der Ansprüche 8 bis 13, **dadurch gekennzeichnet, dass** im Betriebsverfahren die Ist-Konzentration der Abspaltprodukte kontinuierlich oder in festgelegten Zeitabständen mit ihrer im Kalibrierverfahren ermittelten Grenzkonzentration und/oder ihrer Maximalkonzentration verglichen wird und/oder der Messparameter-Istwert kontinuierlich oder in festgelegten Zeitabständen mit seinem im Kalibrierverfahren ermittelten Messparametergrenzwert und/oder seinem Messparametermaximalwert verglichen wird und die Luftwechselrate unter Berücksichtigung der Grenzkonzentration, der Maximalkonzentration, des Messparametergrenzwerts und/oder des Messparametermaximalwerts eingestellt wird.

15. Verfahren nach Anspruch 13 oder 14, **dadurch gekennzeichnet, dass** die Luftwechselrate erhöht wird, wenn die Ist-Konzentration der Abspaltprodukte seine im Kalibrierverfahren ermittelte Grenzkonzentration oder Maximalkonzentration überschreitet und/oder wenn der Messparameter-Istwert seinen im Kalibrierverfahren ermittelten Messparametergrenzwert und/oder Messparametermaximalwert überschreitet.

16. Verfahren nach einem oder mehreren der Ansprüche 8 bis 15, **dadurch gekennzeichnet, dass** im Kalibrierverfahren anhand der Konzentrationsänderung der flüchtigen Abspaltprodukte und/oder anhand der Änderung des Messparameter-Istwerts eine minimale Luftwechselrate und/oder ein optimierter Luftwechselratenverlauf ermittelt wird und im Betriebsverfahren diese minimale Luftwechselrate und/oder der optimierter Luftwechselratenverlauf eingestellt wird.

17. Verfahren nach einem oder mehreren der vorangehenden Ansprüche, **dadurch gekennzeichnet, dass** es folgende Schritte umfasst:
**A) mindestens ein Kalibrierverfahren, umfassend:**
Schritt 1: mindestens ein vorvernetztes Elastomer-Testbauteil wird bei einer vorbestimmten Temperatur thermisch behandelt, wodurch flüchtige Abspaltprodukte aus dem mindestens einen Testbauteil freigesetzt werden;
Schritt 2: mindestens ein Messparameter-Istwert, der mit der Ist-Konzentration der freigesetzten Abspaltprodukte und/oder mit der Änderung der Ist-Konzentration der freigesetzten Abspaltprodukte korreliert, vorzugsweise die Ist-Konzentration der freigesetzten Abspaltprodukte, die Änderung der Ist-Konzentration der freigesetzten Abspaltprodukte, der Ist-Wert des Flächeninhalts einer IR-Absoptionsbande der freigesetzten Abspaltprodukte und/oder der Ist-Wert der Intensität einer IR-Absoptionsbande der freigesetzten Abspaltprodukte, wird kontinuierlich oder in festgelegten Zeitabständen inline und/oder online, vorzugsweise mittels Infrarot-Gasphasen Spektroskopie, bestimmt;
Schritt 3: in festgelegten Zeitabständen wird jeweils mindestens ein Testbauteil der thermischen Behandlung entnommen und es wird der Ist-Wert mindestens eines Performance-Parameters des Testbauteils bestimmt, wobei der Performance-Parameter vorzugsweise mit dem Vernetzungsgrad des Testbauteils, mit dem Restgehalt flüchtiger Bestandteile im Elastomer-Bauteil und/oder dem Restgehalt extrahierbarer Bestandteile korreliert; und wobei der Messparameter-Istwert, der zu dem Zeitpunkt gemessen wird, bei dem ein Testbauteil entnommen wird, das mindestens einen Sollwert des Performance-Parameters des Testbauteils aufweist, dem Messparameter-Sollwert entspricht;
Schritt 4: falls das Betriebsverfahren mit anderen Bedingungen als das Kalibrierverfahren durchgeführt werden soll, vorzugsweise mit anderen Ofenbeladungen, Luftwechselraten, Temperaturverläufen und/oder anderen Temperaturen, wird der in Schritt 3 erhaltene Messparameter-Sollwert gegebenenfalls an die anderen Bedingungen adaptiert;
**B)mindestens ein Betriebsverfahren, umfassend:**
Schritt 1': mindestens ein vorvernetztes Elastomer-Bauteil wird bei einer vorbestimmten Temperatur thermisch behandelt, wodurch flüchtige Abspaltprodukte aus dem Bauteil freigesetzt werden;
Schritt 2': der Messparameter-Istwert wird kontinuierlich oder in festgelegten Zeitabständen inline und/oder online mittels Infrarot-Gasphasen Spektroskopie bestimmt;
Schritt 3': der Messparameter-Istwert wird mit dem im Kalibrierverfahren ermittelten Messparameter-Sollwert verglichen und dieser Abgleich wird zur Einstellung von Verfahrensparametern des Betriebsverfahrens verwendet.

18. Verfahren nach Anspruch 17, **dadurch gekennzeichnet, dass** die Einstellung von Verfahrensparametern des Betriebsverfahrens darin besteht, dass die Temperaturbehandlung des mindestens einen Elastomer-Bauteils, vorzugsweise automatisiert, beendet und/oder die Temperaturbehandlung für einen bestimmten festgelegten Zeitraum weitergeführt und die Temperaturbehandlung, vorzugsweise automatisiert, beendet wird, sobald der Messparameter-Istwert dem Messparameter - Sollwert entspricht oder diesen unter- oder überschreitet, vorzugsweise sobald der Istwert des Flächeninhalts einer IR-Absoptionsbande seinen Sollwert unterschreitet.

19. Verfahren nach einem oder mehreren der vorangehenden Ansprüche, **dadurch gekennzeichnet, dass** das Verfahren folgende Schritte umfasst:
- in einem Kalibrierverfahren wird a) eine Grenzkonzentration der Abspaltprodukte und/oder b) ein Messparametergrenzwert ermittelt, der mit der Grenzkonzentration der Abspaltprodukte korreliert, wobei das Überschreiten der Grenzkonzentration und/oder des Messparametergrenzwerts einen unerwünschten Einfluss auf das Verfahren hat, beispielsweise zur Ausbildung einer entzündbaren Atmosphäre führt und/oder c) eine Maximalkonzentration der Abspaltprodukte ermittelt, die in einem festgelegten Abstand unterhalb der Grenzkonzentration liegt und/oder d) ein Messparametermaximalwert ermittelt wird, der in einem festgelegten Abstand unterhalb des Messparametergrenzwerts liegt;
- in einem Betriebsverfahren wird die Ist-Konzentration der Abspaltprodukte kontinuierlich oder in festgelegten Zeitabständen mit ihrer im Kalibrierverfahren ermittelten Grenzkonzentration und/oder ihrer Maximalkonzentration verglichen und/oder der Messparameter-Istwert wird kontinuierlich oder in festgelegten Zeitabständen mit seinem im Kalibrierverfahren ermittelten Messparametergrenzwert oder Messparametermaximalwert verglichen und die Luftwechselrate wird so eingestellt, dass die Ist-Konzentration der Abspaltprodukte unterhalb der Grenzkonzentration oder der Maximalkonzentration liegt und/oder der Messparameter-Istwert unterhalb des Messparametermaximalwerts und/oder des Messparametergrenzwerts liegt aber die Luftwechselrate dennoch möglichst niedrig ist.

20. Vernetztes Elastomer-Bauteil, hergestellt mit einem Verfahren nach einem oder mehreren der vorangehenden Ansprüche.
